# EUROPEAN PATENT SPECIFICATION

(11) **EP 4 284 470 B1**
(45) Date of publication and mention of the grant of the patent: **21.10.2026**
(21) Application number: 22707906.8
(22) Date of filing: 17.02.2022
(51) Int. Cl.: A61M 5/20, A61M 5/315, A61M 5/32

(54) **MEDICATION ADMINISTRATION DEVICE**
VORRICHTUNG ZUR VERABREICHUNG VON MEDIKAMENTEN
DISPOSITIF D'ADMINISTRATION DE MÉDICAMENT

(30) Priority: 24.02.2021 JP 2021027881
(43) Date of publication of application: 06.12.2023
(73) Proprietor: TERUMO Kabushiki Kaisha, Tokyo 151-0072 (JP)
(72) Inventor: MATSUMOTO, Fumiya, Fujinomiya-shi, Shizuoka 418-0004 (JP); OKIHARA, Hitoshi, Fujinomiya-shi, Shizuoka 418-0004 (JP)
(74) Representative: Casalonga
(86) International application number: PCT/JP2022/006448
(87) International publication number: WO 2022/181451

(56) References cited:
- CA-A1- 2 359 375
- US-A1- 2016 287 801
- US-A1- 2020 282 151

## Description

### Technical Field

The present invention relates to a medication administration device for administering a medication to a living body by puncturing.

### Background Art

In the related art, a portable medication administration device is known for giving a subcutaneous injection of a medication to a user (patient) to be injected by puncturing the user's skin. As disclosed in JP 6154061 B2, such a medication administration device includes, for example, a needle protection sleeve and a piston that are slidably disposed inside a casing, a sleeve-like actuator configured to act on the piston when ejecting a medication, and a first spring configured to act on the actuator. When a distal end of the needle protection sleeve is brought into contact with the skin, the casing moves toward the skin, and a distal end of a needle protrudes from the needle protection sleeve for performing a puncture in the skin. Then, the piston is moved axially by resilience of the first spring to eject the medication in a product container through the needle and subcutaneously administer the medication to the skin.

On completion of the medication administration, a signaler engaged with the actuator is released, and a spring force of a second spring disposed on an outer periphery of the first spring moves the signaler in a direction opposite to a direction of administrating the medication, causing the signaler to abut on a signal stopper disposed at a proximal end of the casing. Accordingly, an auditory or tactile signal is sent, which makes it possible to confirm that the administration of the medication is completed.
Document US 2016/287801 A1 discloses a medicament delivery device. Document CA 2 359 375 A1 discloses an injection device. Document US 2020/282151 A1 discloses an injector device for a medicament.

### Summary of Invention

In a medication administration device as one disclosed in JP 6154061 B2, movement of a piston toward a distal end along with axial movement of a needle protection sleeve acts as a trigger to start administration of a medication or to generate a signal. Therefore, it is desirable that the piston move axially in a stable manner inside a casing, and a diameter of the piston is controlled to suppress radial eccentricity of the piston to the casing.

However, such a piston is typically a molded product formed from a resin material and may vary in diameter due to, for example, shrinkage attributed to the molding and dimension errors of a molding die. Furthermore, there is a limit on highly-accurate control of the diameter, and there is also a concern that the piston rotates circumferentially relative to the casing.

In this manner, when a radial position (eccentricity) of the piston varies with respect to the casing or when the piston circumferentially rotates relative to the casing, the piston is deteriorated in straightness during the axial movement inside the casing, which hinders the piston from moving smoothly. Such a problem may pose obstacles when starting medication administration or generating the signal.

An object of the present invention is to provide a medication administration device that enables stable operation by suppressing eccentricity of a plunger to a casing and by enhancing straightness.

An aspect of the present invention is a medication administration device for administering a medication to a living body, the medication administration device including: a casing having a hollow cylindrical shape; a syringe including a barrel housed in the casing and filled with the medication and a puncture needle communicated with the barrel and configured to administer the medication to the living body; a needle cover having a hollow cylindrical shape, disposed inside the casing, covering a distal end of the syringe, and configured to be displaced toward a proximal end relative to the casing when the needle cover is pressed against a puncture target; a plunger disposed inside the casing, and configured to eject the medication through the puncture needle by moving a distal end of the plunger inside the barrel; and a cap detachably disposed at a distal end of the needle cover, and configured to be detached when the puncture needle punctures the puncture target, in which one of an inner peripheral surface of the casing and an outer peripheral surface of the plunger facing the inner peripheral surface includes a plurality of guide ribs protruding toward the other, the other of the inner peripheral surface of the casing and the outer peripheral surface of the plunger includes a guide groove into which at least a part of the guide ribs is inserted and which extends in a moving direction of the plunger, and the guide ribs are formed axially, the number of the guide ribs being at least three, and the guide ribs including, between each guide rib and the guide groove, a first clearance formed in an extending direction of the guide ribs and a second clearance formed in a direction perpendicular to the extending direction.

According to the present invention, the inside of the casing included in the medication administration device includes the plunger that ejects the medication through the puncture needle when the distal end of the plunger moves into the syringe, the plurality of guide ribs protruding toward the other is disposed on one of the inner peripheral surface of the casing and the outer peripheral surface of the plunger facing the inner peripheral surface, and the guide groove into which at least a part of the guide rib is inserted and which extends along the moving direction of the plunger is disposed on the other of the inner peripheral surface of the casing and the outer peripheral surface of the plunger. At least three guide ribs are disposed axially. Furthermore, the first clearance is formed between each guide rib and the guide groove in the extending direction of the guide ribs, and the second clearance is formed in the direction perpendicular to the extending direction.

For this reason, in the casing, when the plunger rod is moved toward the distal end and ejects the medication through the puncture needle, the plunger rod is guided along the guide ribs inserted in the guide groove, so that the plunger does not rotate relative to the casing and is movable in a straight line toward the distal end. In addition, as compared with a conventional medication administration device that secures the straightness with respect to the casing by controlling a diameter of the piston, it is possible to suppress dimensional tolerances and to suppress the radial eccentricity of the plunger to the casing by reducing widths of the guide ribs and the guide groove.

Accordingly, in the medication administration device, suppressing the eccentricity of the plunger to the casing and enhancing the straightness make it possible to move the plunger smoothly toward the distal end, which enables stable administration of the medication.

### Brief Description of Drawings

[Fig.1]FIG. 1 is a perspective view illustrating an external appearance of a medication administration device according to an embodiment of the present invention;
[Fig.2]FIG. 2 is an exploded perspective view of the medication administration device illustrated in FIG. 1;
[Fig.3]FIG. 3 is an entire cross-sectional view of the medication administration device illustrated in FIG. 1;
[Fig.4]FIG. 4 is an enlarged cross-sectional view illustrating the vicinity of a distal end of the medication administration device in FIG. 3;
[Fig.5]FIG. 5 is an enlarged cross-sectional view illustrating the vicinity of a proximal end of the medication administration device in FIG. 3;
[Fig.6]FIG. 6 is a longitudinal sectional view taken along line VI-VI in FIG. 3;
[Fig.7]FIG. 7 is an entire cross-sectional view illustrating another cross section of the medication administration device in FIG. 3;
[Fig.8]FIG. 8 is an enlarged cross-sectional view illustrating the vicinity of the proximal end of the medication administration device in FIG. 7;
[Fig.9]FIG. 9A is a partially cutaway enlarged perspective view illustrating a state where a proximal end of a plunger rod is inserted in guide grooves of an end guide, and
FIG. 9B is a view for describing actions near a distal end of the plunger rod in FIG. 9A;
[Fig.10]FIG. 10A is a partially cutaway enlarged perspective view illustrating a state where the plunger rod starts to rotate along inclined guiding portions, and FIG. 10B is a view for describing actions near the distal end of the plunger rod in FIG. 10A;
[Fig.11] FIG. 11A is a partially cutaway enlarged perspective view illustrating a state where the plunger rod moves toward the distal end along straight guiding portions, and
FIG. 11B is a view for describing actions near the distal end of the plunger rod in FIG. 11A;
[Fig.12] FIG. 12 is an enlarged cross-sectional view illustrating a state where a cap is pulled toward the distal end of the medication administration device in FIG. 4;
[Fig.13] FIG. 13 is an enlarged cross-sectional view of the cap in FIG. 12, illustrating a state where a proximal end of a holding arm of the cap is inclined radially outward;
[Fig.14] FIG. 14 is an entire cross-sectional view illustrating a state where the cap is detached from the medication administration device illustrated in FIG. 3;
[Fig.15] FIG. 15 is an enlarged cross-sectional view illustrating the vicinity of the distal end of the medication administration device illustrated in FIG. 14;
[Fig.16] FIG. 16 is an enlarged cross-sectional view illustrating the vicinity of the proximal end of the medication administration device in FIG. 14;
[Fig.17] FIG. 17 is an entire cross-sectional view illustrating a state where the medication administration device in FIG. 14 starts to puncture the skin;
[Fig.18] FIG. 18 is an enlarged cross-sectional view illustrating the vicinity of the proximal end of the medication administration device in FIG. 17;
[Fig.19] FIG. 19 is an entire cross-sectional view illustrating a state where a puncture of the skin by the medication administration device in FIG. 17 is completed;
[Fig.20]FIG. 20 is an enlarged cross-sectional view illustrating the vicinity of the proximal end of the medication administration device in FIG. 19;
[Fig.21]FIG. 21 is an entire cross-sectional view illustrating another cross section of the medication administration device in FIG. 19 after the completion of puncture;
[Fig.22]FIG. 22 is an enlarged cross-sectional view illustrating the vicinity of the proximal end of the medication administration device in FIG. 19;
[Fig.23]FIG. 23 is an entire cross-sectional view illustrating a state where the plunger rod starts to move toward the distal end of the medication administration device in FIG. 21;
[Fig.24]FIG. 24 is an enlarged cross-sectional view illustrating the vicinity of the proximal end of the medication administration device in FIG. 23;
[Fig.25]FIG. 25 is an entire cross-sectional view illustrating a state where the plunger rod further moves toward the distal end of the medication administration device in FIG. 19, indicating that the medication administration is completed;
[Fig.26]FIG. 26 is an enlarged cross-sectional view illustrating the vicinity of the proximal end of the plunger rod in the medication administration device in FIG. 25;
[Fig.27]FIG. 27 is an entire cross-sectional view illustrating a state where the plunger rod is further moved toward the distal end of the medication administration device in FIG. 25 after the completion of the medication administration;
[Fig.28]FIG. 28 is an enlarged perspective view illustrating the vicinity of a proximal end of a plunger rod according to a modification in which first and second contact ribs are disposed on outer peripheral surfaces of flexible portions;
[Fig.29]FIG. 29A is an enlarged front view illustrating a state where the proximal end of the plunger rod in FIG. 28 is inserted in guide grooves of an end guide, and FIG. 29B is an enlarged front view illustrating a state where the plunger rod in FIG. 29A is rotated by moving along the guide grooves;
[Fig.30]FIG. 30 is an entire cross-sectional view illustrating a state where a puncture needle moves toward the proximal end and is housed in a cover sleeve of the medication administration device in FIG. 27; and
[Fig.31]FIG. 31 is an enlarged cross-sectional view illustrating the vicinity of the proximal end of the medication administration device in FIG. 30.

### Description of Embodiments

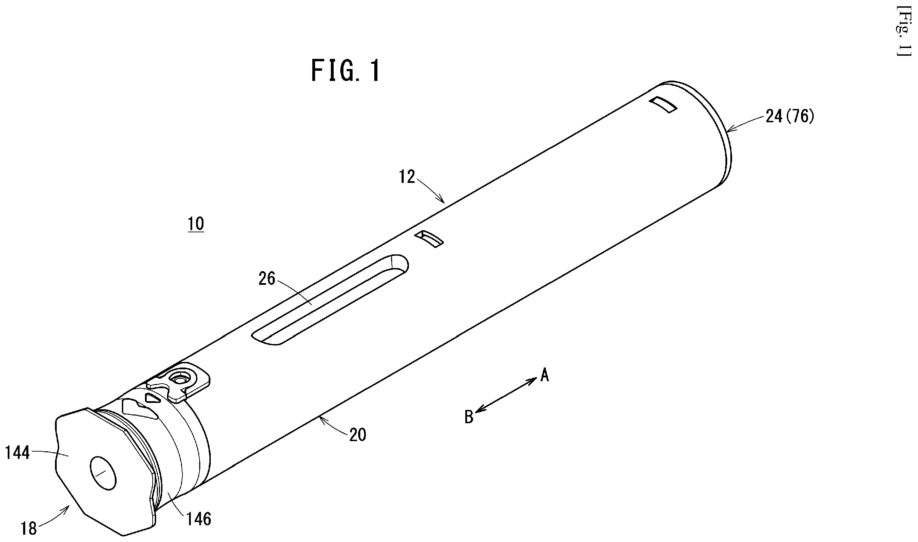

A medication administration device 10 is used, for example, to subcutaneously administer a medication M to a patient as a user. As illustrated in FIGS. 1 to 5, the medication administration device 10 includes a casing 12 having a hollow cylindrical shape, a cover sleeve (needle cover) 14 movably housed in the casing 12, a syringe 16 housed in the cover sleeve 14, and a cap 18 detachably disposed at a distal end of the casing 12.

The casing 12 is formed of, for example, a resin material and includes a cylindrical body 20, a sleeve body 22, and an end cap 24. The cylindrical body 20 is circular in cross section and has outer and inner diameters substantially constant along an axial direction (in directions of arrows A and B). The sleeve body 22 is housed in the cylindrical body 20 on the side closer to a proximal end of the cylindrical body 20 (in direction of arrow A). The end cap 24 closes the proximal end of the cylindrical body 20.

The cylindrical body 20 has a predetermined axial length (in directions of arrows A and B) and has distal and proximal ends both opened. In addition, the cylindrical body 20 has a peripheral wall provided with a viewing window 26 or an opening axially elongated and disposed on the side slightly closer to the distal end than the center of the cylindrical body 20 in the axial direction. The viewing window 26 penetrates the peripheral wall of the cylindrical body 20 and is opened at a position corresponding to a barrel 124 of the syringe 16 housed in the cylindrical body 20, and thus enables a user to check the medication M in the barrel 124.

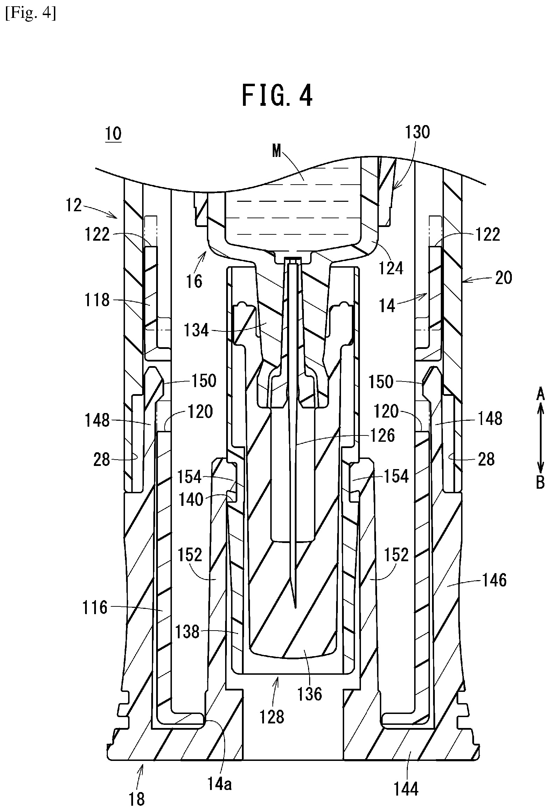

Furthermore, the distal end of the cylindrical body 20 is provided with a pair of recesses 28 at positions facing holding arms 148 (to be described) of the cap 18 (see FIG. 4). The recesses 28 are, for example, rectangular in cross section and are radially recessed into an inner peripheral surface of the cylindrical body 20. The recesses 28 have a predetermined length from the distal end to the proximal end of the cylindrical body 20 (in direction of arrows A and B) and are formed symmetrically about the axis of the cylindrical body 20. The recesses 28 face the holding arms 148 when the cap 18 is mounted on the distal end of the cylindrical body 20.

The sleeve body 22 has a hollow shape and includes a body main part 30 having a cylindrical shape and an expanded diameter portion 32 formed at a proximal end of the body main part 30 (in direction of arrow A). The body main part 30 has a diameter substantially constant along an axial direction (in directions of arrows A and B) and has an inner peripheral surface provide with a plurality of guide ribs 34 protruding radially inward.

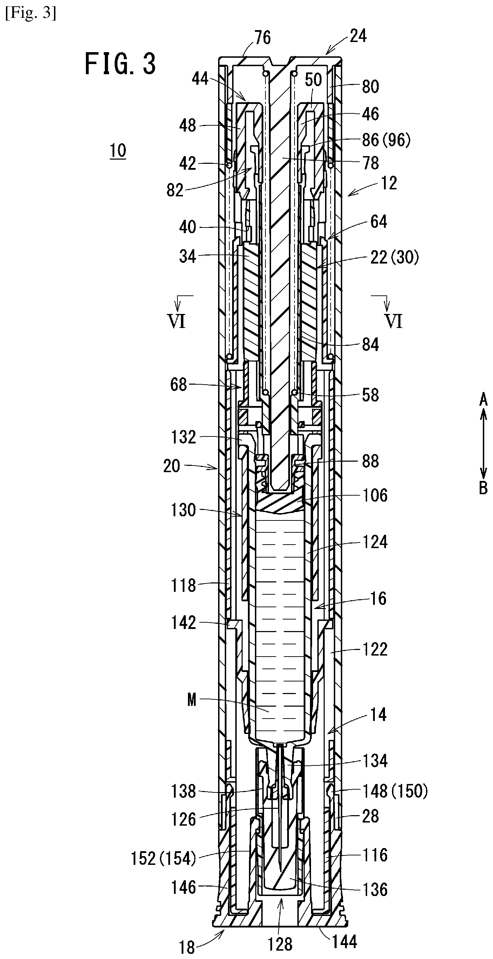

As shown in FIGS. 3, 5, and 6, for example, at least three guide ribs 34 are disposed at regular intervals along a circumferential direction of the body main part 30, having a substantially constant thickness, and protruding radially inward in a linear manner from the inner peripheral surface of the body main part 30. Furthermore, the guide ribs 34 are formed equally to have an equal length protruding radially from the inner peripheral surface. Hereinafter described is a case where four guide ribs 34 are disposed about the axis of the body main part 30 at 90-degree intervals and the guide ribs 34 in pair face each other across the axis of the body main part 30.

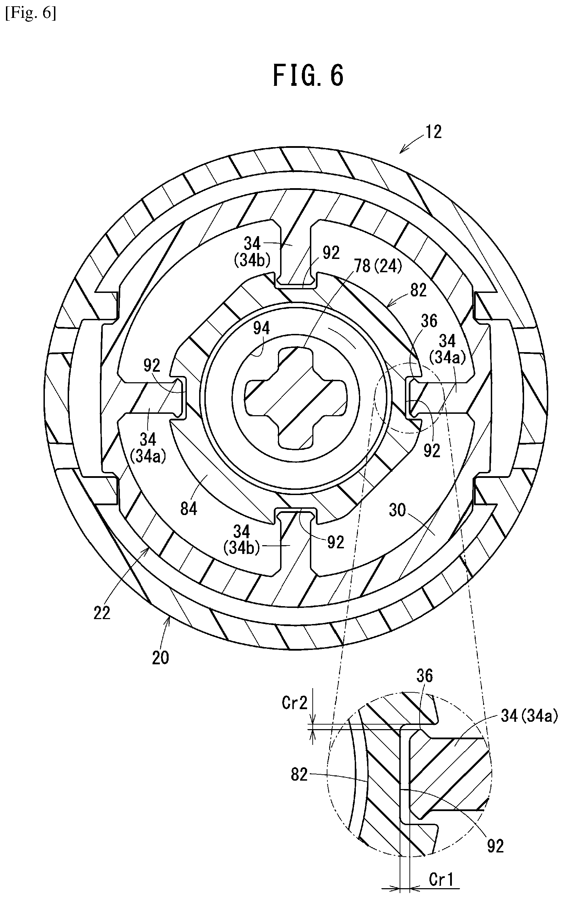

As illustrated in FIG. 6, each of the guide ribs 34 has a radially inner end provided with ridges (protruding portion) 36 protruding in a width direction perpendicular to an extending direction of the guide ribs 34. The ridges 36 are provided at both edges of the inner end in the width direction. Each of the ridges 36 is, for example, triangular in cross section, being gradually tapered toward its apex in the width direction. The guide ribs 34 are engaged with a plunger rod 82 (to be described) to movably guide the plunger rod 82 along the axial direction (in directions of arrows A and B).

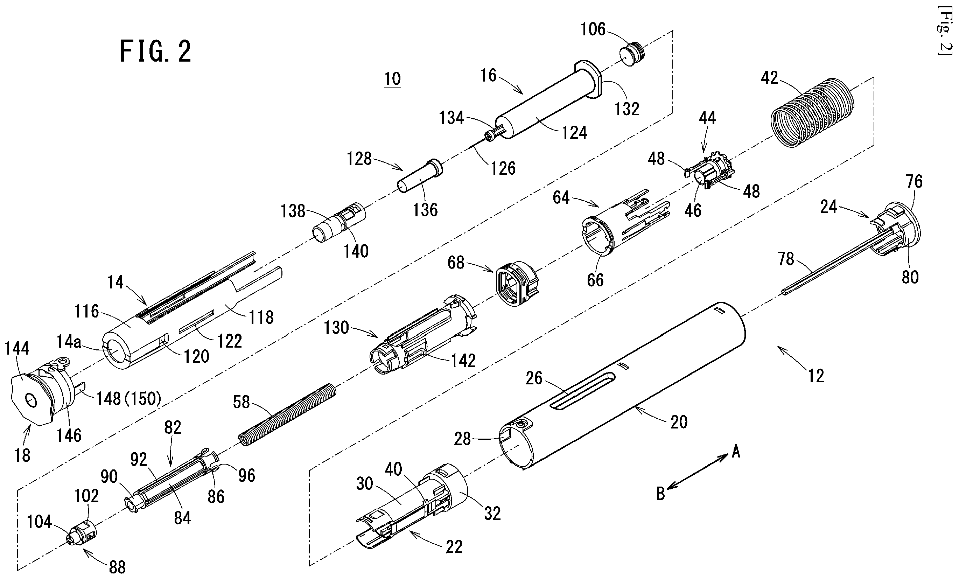

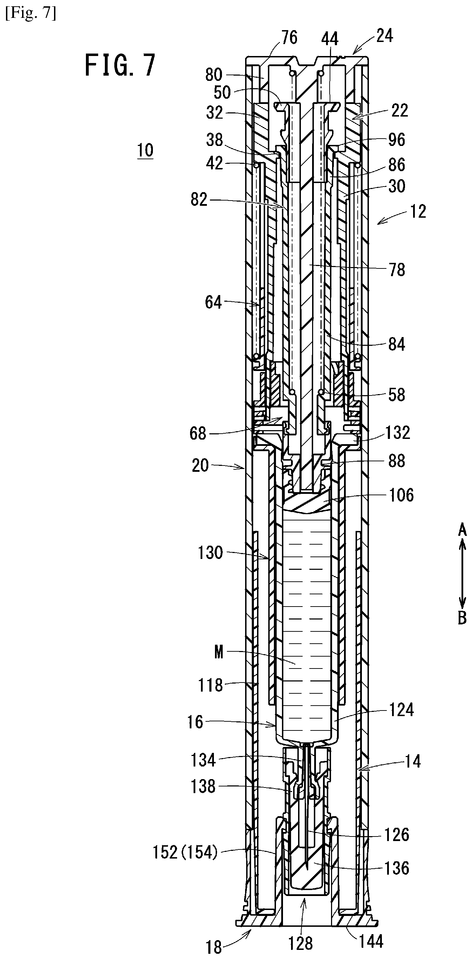

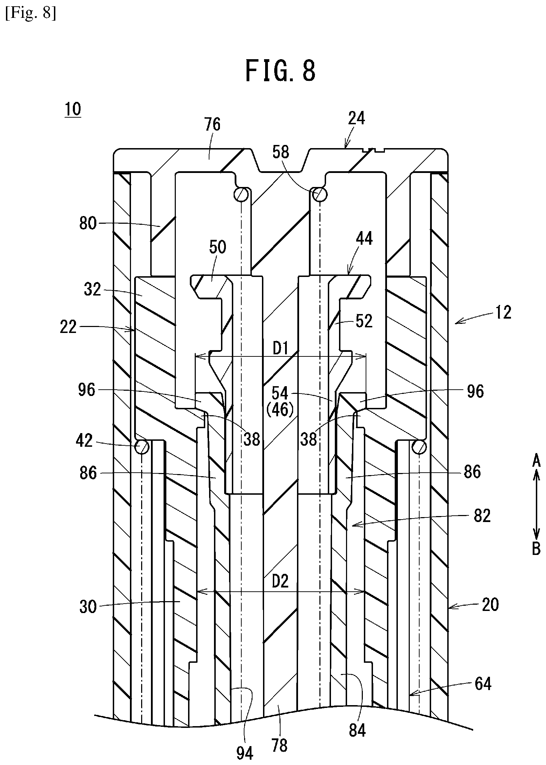

As illustrated in FIGS. 2, 3, and 5, a lock pin 44 and the plunger rod 82 (to be described) are housed in the sleeve body 22, being axially movable. As illustrated in FIGS. 7 and 8, a plurality of protruding portions 38 protruding radially inward is formed at a boundary between the expanded diameter portion 32 and the body main part 30. The protruding portions 38 are rectangular in cross section, having a predetermined height protruding from the inner peripheral surface of the body main part 30. Furthermore, the protruding portions 38 are arcuate in cross section when viewed from an axial direction of the sleeve body 22. Each of the protruding portions 38 has a proximal end face gradually tapered radially inward toward its distal end (in direction of arrow B) (see FIG. 8).

As illustrated in FIGS. 7 and 8, the proximal ends of the protruding portions 38 (in direction of arrow A) are formed in such a manner that collars 96 of a plurality of flexible portions 86 in the plunger rod 82 (to be described) are engaged with the protruding portions 38. The number and positions of the protruding portions 38 are designed according to the number and positions of the flexible portions 86.

The sleeve body 22 is housed in the cylindrical body 20 from the proximal end of the opened cylindrical body 20. The expanded diameter portion 32 of the sleeve body 22 is engaged with an inner peripheral part of the cylindrical body 20, so that the sleeve body 22 is fixed to the cylindrical body 20 while the expanded diameter portion 32 is housed in the cylindrical body 20 in such a manner that a proximal end of the expanded diameter portion 32 is placed closer to the distal end of the cylindrical body 20 (in direction of arrow B) than the proximal end of the cylindrical body 20 by a predetermined distance.

On the other hand, as illustrated in FIGS. 2, 3, and 5, the body main part 30 has an outer peripheral surface provided with a pair of lock grooves 40 that are recessed radially inward. The lock grooves 40 are formed symmetrically about the axis of the sleeve body 22. The lock grooves 40 are engageable with lock claws 62 of the lock pin 44 (to be described).

Furthermore, as a sleeve spring 42, a coil spring is inserted between the outer peripheral surface of the body main part 30 and the inner peripheral surface of the cylindrical body 20. The sleeve spring 42 has a proximal end engaged with a distal end of the expanded diameter portion 32 and a distal end engaged with a proximal end of a slide sleeve 64 (to be described). The sleeve spring 42 biases the slide sleeve 64 in a direction away from the sleeve body 22, that is, toward the distal end (in direction of arrow B).

Still further, the lock pin 44 is housed in the sleeve body 22, straddling the expanded diameter portion 32 and the body main part 30.

The lock pin 44 is disposed inside the sleeve body 22, being axially movable (in directions of arrows A and B). The center of the lock pin 44 is provided with a cylindrical pin body 46 that extends axially. An outer periphery of the pin body 46 is provided with a pair of arms 48 connected to a proximal end of the pin body 46 in an integrated manner.

The pin body 46 includes an end wall 50, a large diameter portion 52, and a small diameter portion 54. The end wall 50 is a flat wall perpendicular to an axial direction and disposed at the proximal end of the pin body 46. The large diameter portion 52 extends from the end wall 50 toward the distal end (in direction of arrow B). The small diameter portion 54 is disposed at a distal end of the large diameter portion 52 (in direction of arrow B) and has a reduced outer diameter. On the axis of the pin body 46, a hole 56 is formed, penetrating the end wall 50, the large diameter portion 52, and the small diameter portion 54 in a straight line. The hole 56 has a constant diameter and extends along the axial direction. In this hole 56, a shaft 78 of the end cap 24 and an injection spring 58 (to be described) are inserted.

The arms 48 each have a proximal end connected to the end wall 50 of the pin body 46. The arms 48 are formed on the outer periphery of the pin body 46, being in parallel to the pin body 46 at a predetermined interval, and having an equal length extending toward the distal end (in direction of arrow B). The arms 48 each have a distal end provided with an engagement end 60 which is engaged with the proximal end of the slide sleeve 64 (to be described) and a lock claw 62 formed on a radially inner side of the engagement end 60.

The engagement ends 60 have a flat shape, being perpendicular to an extending direction of the arms 48. The lock claws 62 protrude radially inward from the engagement ends 60 toward the distal end (in direction of arrow B), being triangular in cross section and tapered toward the distal end.

Inside the cylindrical body 20, the cylindrical slide sleeve 64 is disposed on the side closer to a distal end of the lock pin 44 (in direction of arrow B).

The slide sleeve 64 is disposed inside the cylindrical body 20, being axially movable (in directions of arrows A and B). In the slide sleeve 64, the plunger rod 82 and the body main part 30 of the sleeve body 22 are inserted. A distal end of the slide sleeve 64 includes a catcher 66 having a diameter enlarged radially outward. The catcher 66 holds the distal end of the sleeve spring 42 disposed on an outer periphery of the slide sleeve 64. Due to resilience of the sleeve spring 42, the slide sleeve 64 is biased toward the distal end (in direction of arrow B) with respect to the end cap 24. On the other hand, the proximal end of the slide sleeve 64 is engageable with the engagement ends 60 of the lock pin 44 disposed at the proximal end (in direction of arrow A).

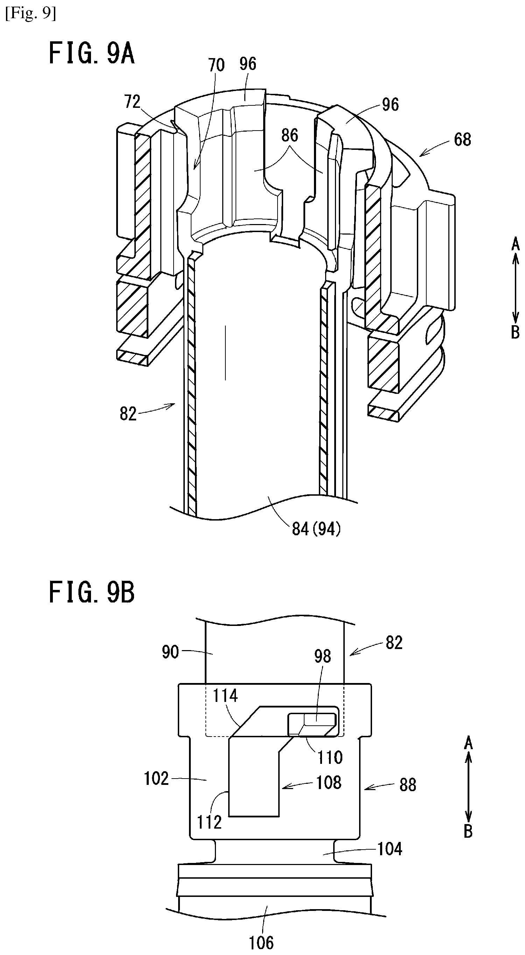

In addition, an end guide 68 faces the distal end of the slide sleeve 64. The end guide 68 has a cylindrical shape, being substantially equal to the slide sleeve 64 in diameter. The plunger rod 82 is inserted in the end guide 68, being axially movable. The end guide 68 has an inner peripheral surface provided with guide grooves 70 (see FIGS. 9A, 10A, and 11A) that allow the plunger rod 82 to rotate.

As illustrated in FIGS. 9A, 10A, and 11A, the guide grooves 70 are recessed radially outward from the inner peripheral surface of the end guide 68. In the guide grooves 70, the collars 96 of the flexible portions 86 in the plunger rod 82 (to be described) are inserted. For example, four guide grooves 70 are disposed at regular circumferential intervals corresponding to the number and positions of the flexible portions 86.

Each of the guide grooves 70 includes an inclined guiding portion 72 and a straight guiding portion 74. The inclined guiding portions 72 are inclined relative to an axial direction of the end guide 68 (in directions of arrows A and B) toward the distal end (in direction of arrow B). The straight guiding portions 74 extend toward the distal end in a straight line from distal ends of the inclined guiding portions 72, and the straight guiding portions 74 have a circumferential width substantially equal to or slightly larger than a width of the collars 96 of the plunger rod 82 (to be described).

As illustrated in FIGS. 2, 3, and 5, the end cap 24 includes a lid 76 that closes the proximal end of the cylindrical body 20 and the shaft 78 that extends from the center of the lid 76 toward the distal end (in direction of arrow B). The lid 76 has a disk shape, being equal to the cylindrical body 20 in outer diameter.

The lid 76 includes a stopper 80 protruding from an end face on the distal end side. The stopper 80 has an annular shape having a predetermined height protruding from the end face. The stopper 80 abuts on the proximal end of the expanded diameter portion 32 of the sleeve body 22 to restrict the movement of the sleeve body 22 toward the proximal end (in direction of arrow A) and hold the sleeve body 22 housed in the cylindrical body 20.

The shaft 78 includes a shaft body extending axially toward the distal end (in direction of arrow B), being housed in the cylindrical body 20 and the sleeve body 22. The shaft 78 extends axially to the vicinity of the center of the cylindrical body 20. The shaft 78 is inserted in a coil spring as the injection spring 58 and the plunger rod 82 in such a manner that the injection spring 58 and the plunger rod 82 are placed on an outer periphery of the shaft 78. The injection spring 58 has an elongated shape corresponding to an axial length of the shaft 78, being interposed between the plunger rod 82 (to be described) and the end face of the lid 76, and being configured to bias the plunger rod 82 toward the distal end.

When the end cap 24 is mounted on the proximal end of the cylindrical body 20, the opened proximal end is closed by the disk-shaped lid 76, and the shaft 78 is placed on the axis of the cylindrical body 20.

As illustrated in FIGS. 2, 3, and 5 to 8, the plunger rod (plunger) 82 includes a rod body 84, the plurality of flexible portions 86, and a mount 90. The rod body 84 has a cylindrical shape elongated in an axial direction (in directions of arrows A and B) and has a constant diameter along the axial direction. The flexible portions 86 are disposed at a proximal end of the rod body 84 (in direction of arrow A) and divided circumferentially. The mount 90 is disposed at a distal end of the rod body 84 (in direction of arrow B). On the mount 90, a top 88 (to be described) is mounted.

The rod body 84 is circular in cross section. The rod body 84 has an outer peripheral surface provided with a plurality of slide grooves (guide grooves) 92 recessed radially inward. In the slide grooves 92, the guide ribs 34 of the sleeve body 22 are inserted. As illustrated in FIG. 6, for example, the slide grooves 92 are rectangular in cross section, being recessed into the outer peripheral surface and disposed at regular circumferential intervals corresponding to the number and positions of the guide ribs 34. Hereinafter described is a case where the rod body 84 is provided with four slide grooves 92 corresponding to the guide ribs 34.

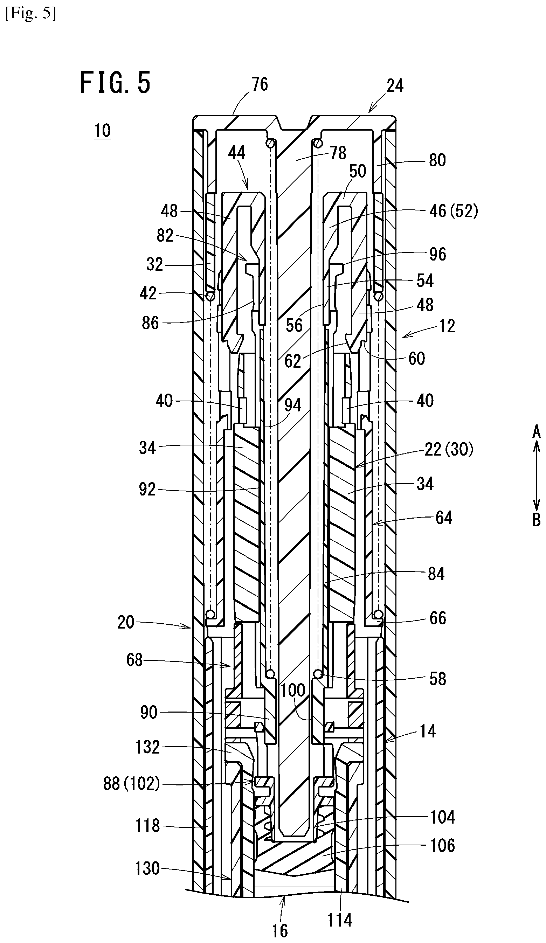

When the plunger rod 82 is housed in the sleeve body 22, distal ends of the guide ribs 34 are inserted in the slide grooves 92 as illustrated in FIGS. 5 and 6. As illustrated in FIG. 6, between each slide groove 92 and the distal end of each guide rib 34, a first clearance Cr1 is formed in the extending direction of the guide ribs 34, and a second clearance Cr2 is formed in a direction perpendicular to the extending direction. The second clearance Cr2 is a clearance between the ridge 36 of each guide rib 34 and an inner surface of each slide groove 92. In other words, the first clearance Cr1 and the second clearance Cr2 are clearances perpendicular to each other.

As illustrated in FIGS. 3 and 5 to 8, the inside of the rod body 84 is provided with a first rod hole 94 that extends axially (in directions of arrows A and B). In the first rod hole 94, the shaft 78 of the end cap 24 and the injection spring 58 are inserted.

The flexible portions 86 have a predetermined length axially protruding (in direction of arrow A) from the proximal end of the rod body 84, being circumferentially separated from each other so as to be disposed between two adjacent slide grooves 92. Furthermore, the flexible portions 86 each have a proximal end (in direction of arrow A) being radially inclinable, using a distal end (in direction of arrow B) connected to the rod body 84 as a fulcrum. The proximal ends of the flexible portions 86 are provided with the collars 96 protruding radially outward. The collars 96 each have a distal end face (in direction of arrow B) being tapered toward the proximal end and inclined gradually radially outward (see FIG. 8).

In addition, the flexible portions 86 have a diameter expanding radially outward from the rod body 84, and the inner side of the flexible portions 86 is formed in such a manner that the small diameter portion 54 of the lock pin 44 is inserted therein. In other words, the flexible portions 86 have an inner diameter larger than that of the first rod hole 94.

Furthermore, as illustrated in FIG. 8, the collars 96 of the flexible portions 86 have an outer diameter D1 slightly larger than an inner diameter D2 of the body main part 30 of the sleeve body 22 (D1 > D2).

As illustrated in FIGS. 2, 3, 5, and 7, the mount 90 has a diameter smaller than the rod body 84. As illustrated in FIGS. 9B, 10B, and 11B, the mount 90 has an outer peripheral surface provided with a pair of engagement sections 98 protruding radially outward and engaged with the top 88 (to be described). The engagement sections 98 extend in a horizontal direction perpendicular to an axial direction of the plunger rod 82, being placed symmetrically about the axis of the plunger rod 82.

As illustrated in FIG. 5, the inside of the mount 90 is provided with a second rod hole 100 that extends axially (in directions of arrows A and B). The second rod hole 100 has a diameter smaller than the first rod hole 94. In the second rod hole 100, the shaft 78 of the end cap 24 is inserted. A boundary between the second rod hole 100 and the first rod hole 94 is a tier with which a distal end of the injection spring 58 is engaged. With such a configuration, the plunger rod 82 is biased by resilience of the injection spring 58 in a direction away from the end cap 24, that is, toward the distal end (in direction of arrow B).

As illustrated in FIGS. 2, 3, 5, 7, 9B, 10B, and 11B, the top 88 is formed into a cup-like shape, having a proximal end opened (in direction of arrow A), and including a cylindrical cup portion 102 and an attacher 104. The cup portion 102 is rotatable relative to the mount 90 of the plunger rod 82, being disposed at the proximal end of the top 88. The attacher 104 protrudes from a distal end of the cup portion 102 toward the distal end (in direction of arrow B). On the attacher 104, a gasket 106 including an elastic material is mounted to cover an outer periphery of the attacher 104.

The mount 90 of the plunger rod 82 is inserted in a proximal end of the cup portion 102. The cup portion 102 has an outer peripheral surface provided with a pair of leading grooves 108 radially penetrating the cup portion 102. In the leading grooves 108, the engagement sections 98 of the plunger rod 82 are inserted.

The leading grooves 108 are formed in pair, being symmetric about the axis of the cup portion 102, and each having a substantially L shape including a horizontal portion 110 and a perpendicular portion 112. The horizontal portions 110 are disposed closer to the proximal end of the cup portion 102, extending along a circumferential direction. The perpendicular portions 112 are perpendicular to the horizontal portions 110, extending from one end in the circumferential direction toward the distal end (in direction of arrow B). In a connection part between each horizontal portion 110 and each perpendicular portion 112, an inclined portion 114 is formed, being inclined at a predetermined angle.

The mount 90 of the plunger rod 82 is inserted in the cup portion 102 and the engagement sections 98 are engaged with the pair of leading grooves 108. Accordingly, the top 88 is axially connected to the plunger rod 82. The top 88 and the plunger rod 82 are axially movable in an integrated manner and are inserted in the barrel 124 of the syringe 16 (to be described) while the gasket 106 is mounted on the attacher 104.

As illustrated in FIGS. 2 to 5 and 7, the cover sleeve 14 is movably disposed inside the cylindrical body 20 included in the casing 12, and the cover sleeve 14 includes a cylindrical sleeve body 116 disposed at a distal end of the cover sleeve 14 (in direction of arrow B) and a pair of cover portions 118 extending from the sleeve body 116 toward the proximal end (in direction of arrow A). The center of the sleeve body 116 is provided with an open hole 14a axially penetrating the sleeve body 116. The cover portions 118 are formed symmetrically about the axis of the cover sleeve 14, having a predetermined length in the axial direction.

In the pair of cover portions 118, engagement holes 120 are opened on the side closer to the sleeve body 116 (in direction of arrow B). With the engagement holes 120, the holding arms 148 of the cap 18 are engaged. Each of the engagement holes 120 has a proximal end (in direction of arrow A) provided with a syringe guide hole 122 that is opened and axially elongated.

The engagement holes 120 have, for example, a rectangular shape elongated in a direction perpendicular to an axial direction of the cover sleeve 14, radially penetrating the cover sleeve 14. The syringe guide holes 122 radially penetrating the cover sleeve 14 are formed axially in a linear manner. With the syringe guide holes 122, a syringe holder 130 (to be described) is axially engaged in a movable manner. In other words, the syringe guide holes 122 have a function of guiding the syringe holder 130 axially.

The syringe 16 includes the hollow barrel 124 filled with the medication M, the gasket 106 slidably inserted in the barrel 124, a puncture needle 126 disposed at a distal end of the barrel 124 and protruding toward the distal end (in direction of arrow B), and a protective cover 128 attached to the distal end of the barrel 124. The barrel 124 is held by the cylindrical syringe holder 130 disposed on an outer periphery of the barrel 124. The medication M to be used is, for example, one used for subcutaneous injection of a patient.

The barrel 124 is a hollow body having a substantially cylindrical shape, including an opening at a proximal end. The proximal end has an outer peripheral part provided with a flange 132 protruding radially outward. The distal end of the barrel 124 is provided with a needle holder 134. The needle holder 134 has a diameter smaller than the barrel 124, protruding in the distal direction and configured to hold a proximal end of the puncture needle 126.

The barrel 124 includes, for example, a transparent resin material and allows a user to visually check the remaining dose of the medication M with which the inside of the barrel 124 is filled from the outside through the viewing window 26 of the casing 12. The flange 132 of the barrel 124 is engaged with a proximal end of the syringe holder 130. Accordingly, the outer periphery of the barrel 124 is covered with the syringe holder 130, and the barrel 124 is held by the syringe holder 130 in an integrated manner without moving axially relative to the syringe holder 130.

The gasket 106 includes, for example, an elastic material such as rubber and is inserted in the barrel 124 through the opened proximal end while being mounted on the attacher 104 of the top 88. Furthermore, the gasket 106 is axially slidable along an inner peripheral surface of the barrel 124. The gasket 106 is inserted in the barrel 124. Accordingly, the proximal end of the barrel 124 is sealed in a liquid-tight manner, and the medication M is enclosed inside the barrel 124.

The puncture needle 126 is a hollow body including a channel through which the medication M flows. The puncture needle 126 protrudes from the needle holder 134 in the distal direction, and the channel communicates with the inside of the barrel 124 filled with the medication M. The medication M with which the inside of the barrel 124 is filled is ejected from a distal end of the puncture needle 126 and administered to a patient.

As illustrated in FIGS. 2 to 4, the protective cover 128 covers the puncture needle 126 by being mounted on the distal end of the barrel 124. The protective cover 128 includes a needle shield 136 and an outer cover 138. The needle shield 136 includes an elastic material such as rubber and is mounted on the needle holder 134. The outer cover 138 further covers an outer periphery of the needle shield 136. A proximal end of the needle shield 136 is mounted on the needle holder 134 to cover the puncture needle 126, and the outer cover 138 is slidably fitted in an outer peripheral surface of the needle shield 136. The outer cover 138 has an outer peripheral surface provided with an annular groove 140 recessed radially inward. With the annular groove 140, holding sections 152 of the cap 18 (to be described) are engaged.

The syringe holder 130 that holds the syringe 16 is provided with a pair of projections 142 protruding radially outward from an outer peripheral surface. The projections 142 are inserted in the syringe guide holes 122 of the cover sleeve 14, and the syringe holder 130 is held inside the cover sleeve 14 together with the syringe 16, being axially movable (in directions of arrows A and B).

While the syringe 16 is held by the syringe holder 130 and housed in the cover sleeve 14, the puncture needle 126 comes closer to the distal end (in direction of arrow B), and the barrel 124 faces the opened viewing window 26 of the casing 12, allowing a user to check the remaining dose of the medication M in the barrel 124 from the outside.

As shown in FIGS. 1 to 4 and 12, the cap 18 has a cylindrical shape with a bottom, including a bottom wall 144 at a distal end of the cap 18 and an annular peripheral wall 146 erected from the bottom wall 144 toward the proximal end (in direction of arrow A). A proximal end of the cap 18 is opened and provided with a pair of holding arms 148 protruding axially (in direction of arrow A) from a proximal end of the peripheral wall 146.

As illustrated in FIGS. 2 to 4, 12, and 13, each of the holding arms 148 has a predetermined width in a circumferential direction of the cap 18, being disposed on a radially inner side of the peripheral wall 146 and radially inclinable using a connection part with the peripheral wall 146 as a fulcrum. A proximal end of each of the holding arms 148 is provided with an outer hook 150 protruding radially inward. Furthermore, the pair of holding arms 148 is placed symmetrically about the axis of the cap 18.

When the cap 18 is mounted on the distal end of the casing 12, the proximal end of the peripheral wall 146 abuts on the distal end of the cylindrical body 20, and the pair of holding arms 148 is inserted in gaps between the cylindrical body 20 and the cover sleeve 14 and placed at positions facing the recesses 28 and the engagement holes 120. Accordingly, the outer hooks 150 of the holding arms 148 are engaged with the engagement holes 120.

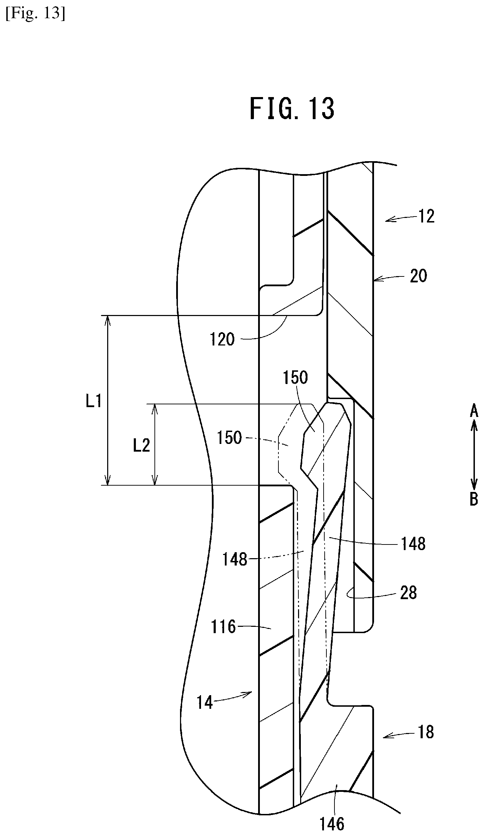

As illustrated in FIG. 13, an axial length L1 of the engagement holes 120 is larger than an axial length L2 of the outer hooks 150 (L1 > L2). In other words, the outer hooks 150 are engaged with the engagement holes 120, being axially movable (in directions of arrows A and B).

As illustrated in FIG. 4, when the cap 18 is mounted on the distal end of the cover sleeve 14, the recesses 28 are displaced from the engagement holes 120 toward the distal end (in direction of arrow B), and the proximal ends of the holding arms 148 provided with the outer hooks 150 are placed closer to the proximal end (in direction of arrow A) than proximal ends of the recesses 28.

Furthermore, four holding sections 152 are disposed inside the cap 18, protruding from a central portion of the bottom wall 144 toward the proximal end (in direction of arrow A). The holding sections 152 have a substantially cylindrical shape circumferentially divided from each other. The outer cover 138 of the protective cover 128 included in the syringe 16 is inserted in the holding sections 152. Accordingly, inner hooks 154 disposed at proximal ends of the holding sections 152 and protruding radially inward are engaged with the annular groove 140 of the outer cover 138.

With such a configuration, through the holding sections 152, the cap 18 is engaged with the protective cover 128 and engaged with the distal end of the cover sleeve 14. Accordingly, the cap 18 is mounted to cover the distal ends of the cover sleeve 14 and the casing 12 while holding the protective cover 128.

The basic configuration of the medication administration device 10 according to the embodiment of the present invention has been described. Hereinafter described are functions and effects of the medication administration device 10.

First, suppose that the unused medication administration device 10 with the cap 18 mounted thereon is accidentally dropped on the floor or the like with the cap 18's side down (from the distal end side).

In the medication administration device 10 illustrated in FIG. 3, for example, when a shock caused by a contact with the floor is propagated to the cover sleeve 14, the cover sleeve 14 may move relative to the casing 12 from a predetermined position toward the proximal end (in direction of arrow A) (see shapes enclosed by dash-dot-dot-lines in FIG. 4). In this case, along with the movement of the cover sleeve 14, distal parts of the engagement holes 120 and the outer hooks 150 of the holding arms 148 come into contact with each other, causing the proximal ends of the holding arms 148 including the outer hooks 150 to be pressed radially outward. However, the recesses 28 are not disposed on radially outer sides of the proximal ends of the holding arms 148, and the radially outward inclination of the holding arms 148 is restricted by the inner peripheral surface of the cylindrical body 20.

Due to this configuration, even when the medication administration device 10 is dropped on the floor or the like with the cap 18's side down and a shock is applied to the cover sleeve 14 toward the proximal end (in direction of arrow A), causing the cover sleeve 14 to move toward the proximal end, the holding arms 148 (outer hooks 150) are reliably kept engaged with the engagement holes 120. Accordingly, the cap 18 is prevented from falling off the distal ends of the casing 12 and the cover sleeve 14, and the cap 18 is reliably held while covering the distal end of the casing 12. Therefore, the puncture needle 126 housed in the casing 12 and the cover sleeve 14 is prevented from protruding accidentally and from being exposed to the outside.

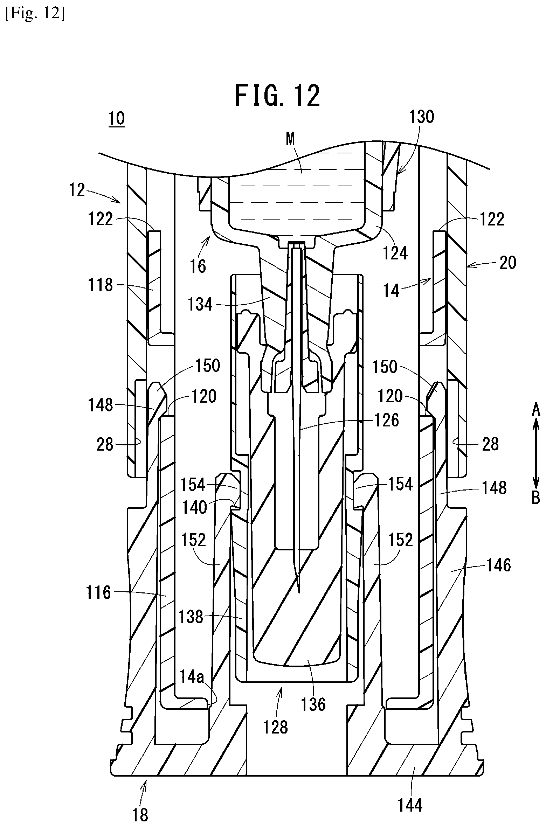

Next, suppose that medication administration is performed with the medication administration device 10. The cap 18 mounted on the distal ends of the casing 12 and the cover sleeve 14 is detached from the unused medication administration device 10 illustrated in FIGS. 3 and 4. In this case, a patient grips the cylindrical body 20 of the casing 12 and pulls the cap 18 in a direction away from the casing 12 (in direction of arrow B), separating the cap 18 from the distal end of the cover sleeve 14 as illustrated in FIG. 12, which causes the outer hooks 150 to move toward the distal end (in direction of arrow B) inside the engagement holes 120.

Accordingly, as illustrated in FIGS. 12 and 13, the outer hooks 150 face the recesses 28. When the cap 18 is further pulled toward the distal end (in direction of arrow B), the holding arms 148 are inclined radially outward under action of contact between the outer hooks 150 and the distal ends of the engagement holes 120, and the proximal ends of the holding arms 148 including the outer hooks 150 move into the recesses 28. This action releases the holding arms 148 engaged with the engagement holes 120. Then, the holding arms 148 move toward the distal end through gaps between the recesses 28, the cylindrical body 20, and the cover sleeve 14.

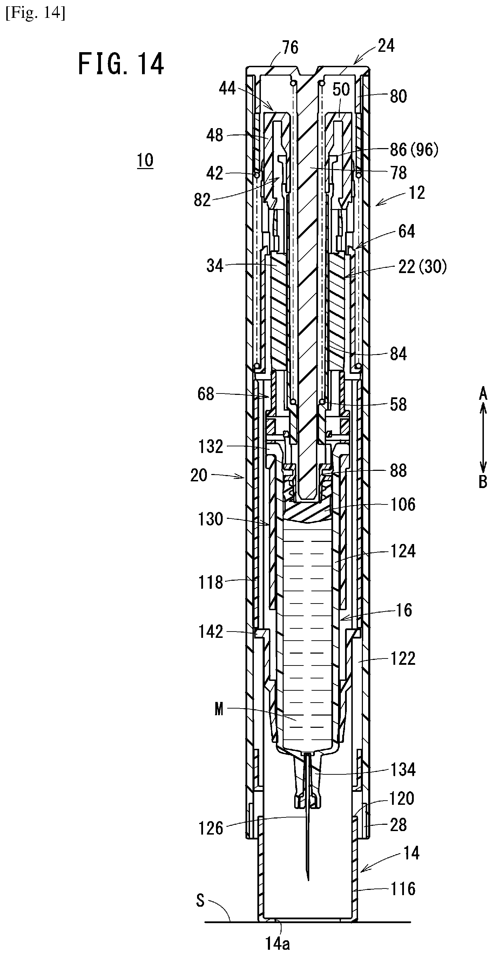

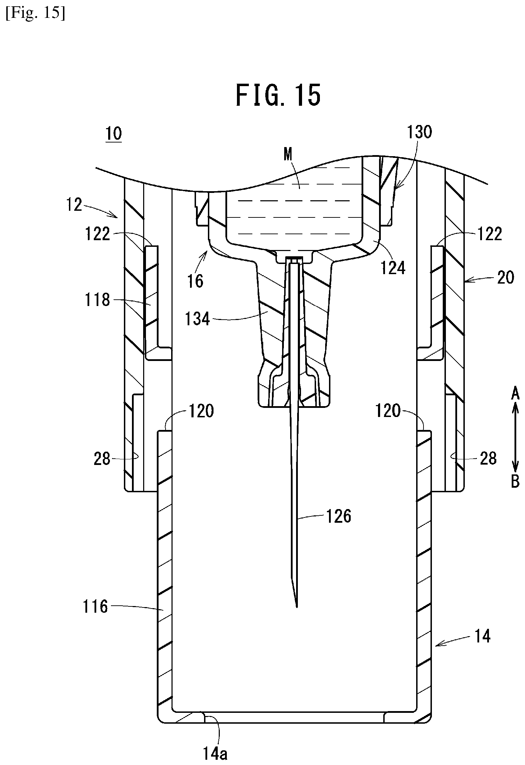

Along with the movement of the cap 18 toward the distal end, the protective cover 128 held by the holding sections 152 moves together with the cap 18, and the protective cover 128 is detached from the needle holder 134 of the barrel 124 as illustrated in FIGS. 14 and 15. In other words, the protective cover 128 of the syringe 16 is detached simultaneously with the detachment of the cap 18 from the distal end of the cover sleeve 14.

Furthermore, the cap 18 and the cover sleeve 14 engaged by the holding arms 148 are released to completely detach the cap 18 from the distal end of the cover sleeve 14. Accordingly, the distal ends of the casing 12 and the cover sleeve 14 are opened, and the protective cover 128 covering the puncture needle 126 is detached concurrently as illustrated in FIGS. 14 and 15.

Next, the medication M is administered with the medication administration device 10 from which the cap 18 is detached as described above.

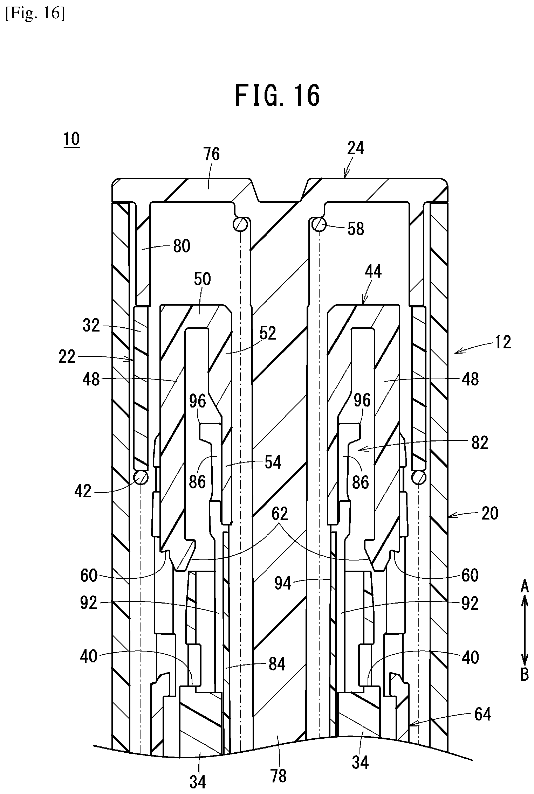

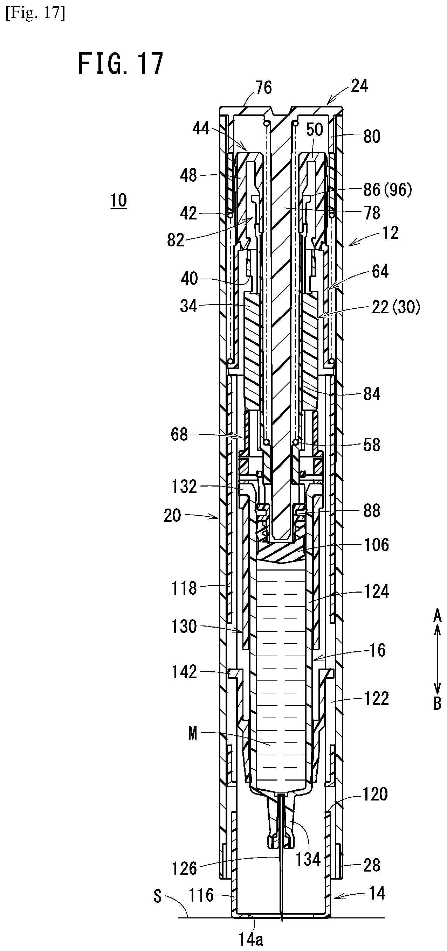

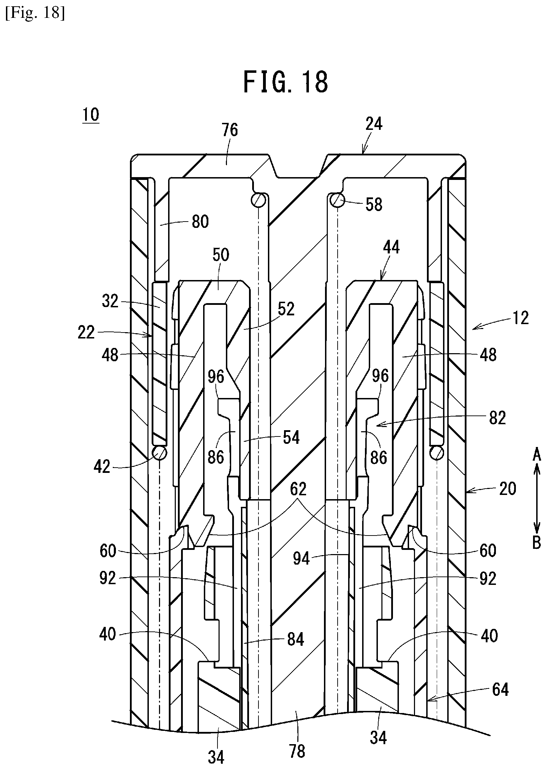

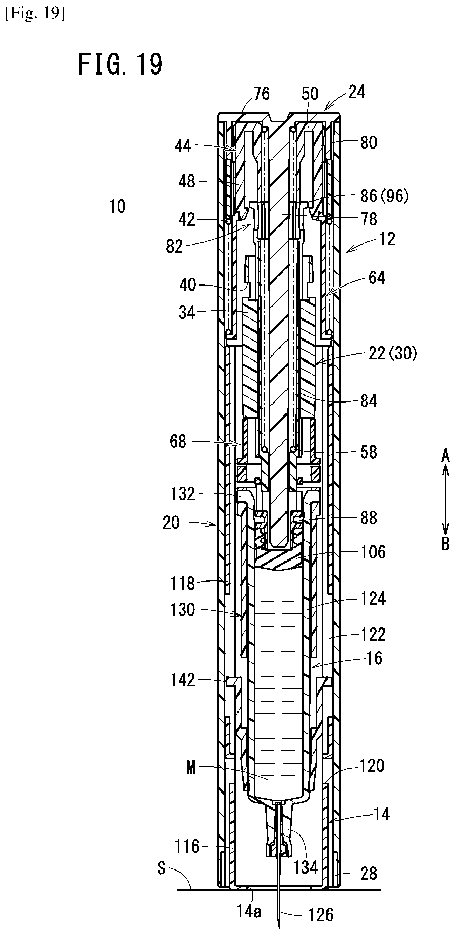

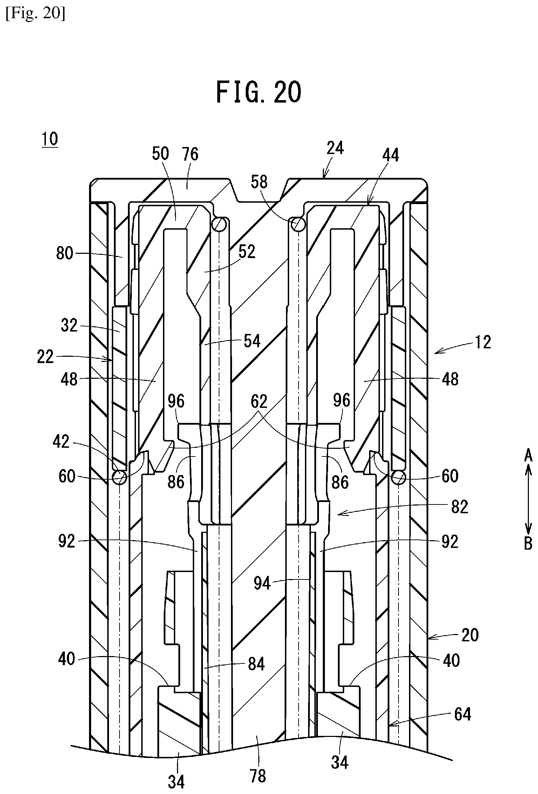

First, while the patient grips the casing 12 of the medication administration device 10 illustrated in FIGS. 14 to 16, the patient presses the distal end of the cover sleeve 14 protruding from the distal end of the casing 12 against his/her skin S or a desired puncture site at a substantially right angle. Then, as illustrated in FIGS. 17 and 18, the patient pushes the casing 12 against the skin S (toward the distal end, in direction of arrow B). Accordingly, as illustrated in FIGS. 19 and 20, the casing 12 moves toward the distal end (in direction of arrow B) relative to the cover sleeve 14 while compressing the sleeve spring 42 toward the distal end.

Since the sleeve body 22 abuts on a proximal end of the cover sleeve 14, the sleeve body 22 and the lock pin 44 do not move relative to each other. Similarly, since the lock pin 44 abuts on the proximal end of the sleeve body 22, the lock pin 44 does not move toward the distal end.

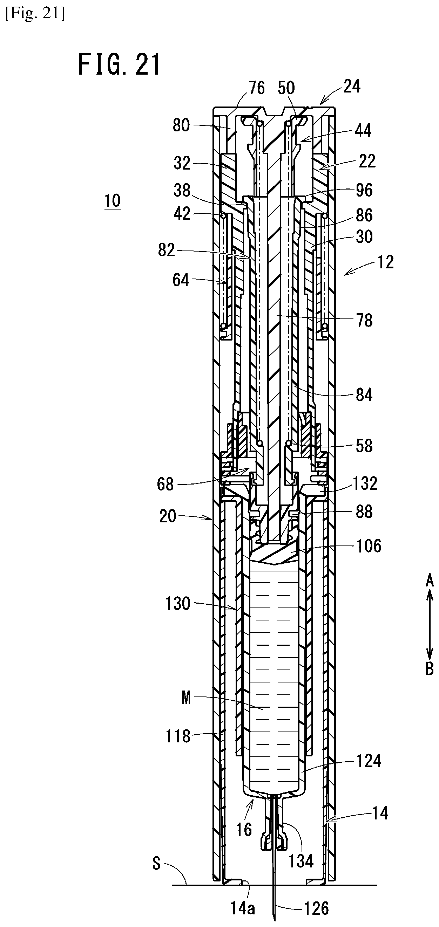

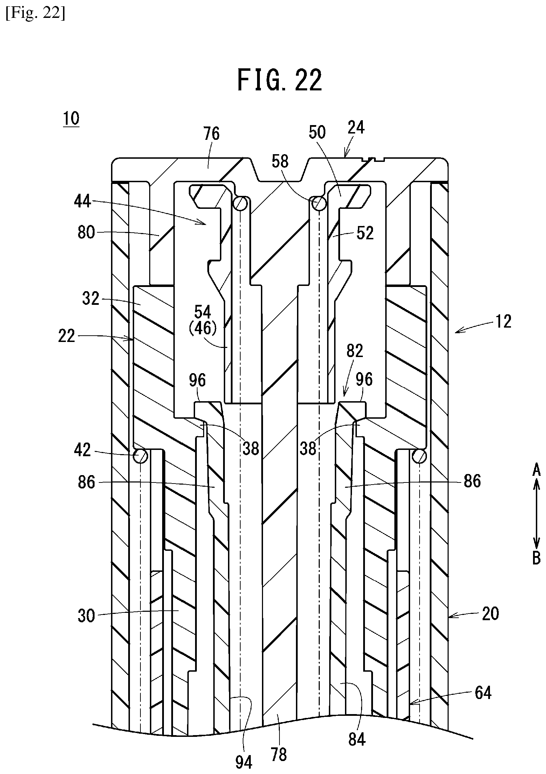

As illustrated in FIG. 19, the puncture needle 126 of the syringe 16 is exposed from a hole 14a of the cover sleeve 14 toward the distal end (in direction of arrow B) to puncture the skin S, and the puncture needle 126 is inserted to a predetermined depth. At this time, as illustrated in FIGS. 21 and 22, the small diameter portion 54 of the lock pin 44 is inserted in the flexible portions 86, and the radially inward inclination of the plunger rod 82 is controlled, so that the plunger rod 82 is kept engaged with the protruding portions 38 of the sleeve body 22. Since the movement relative to the casing 12 is restricted, the plunger rod 82 moves together with the casing 12 in an integrated manner. In other words, the medication M is not yet administered by the plunger rod 82.

Pushing the casing 12 against the skin S in this manner moves the distal end of the cylindrical body 20 to a position substantially equal to the distal end of the cover sleeve 14 as illustrated in FIG. 21, and the puncture needle 126 punctures the skin S to a predetermined depth, thereby completing the puncture.

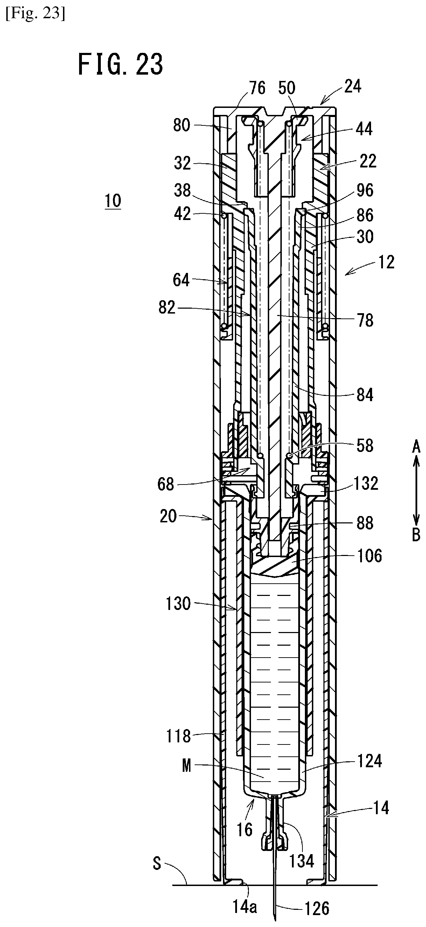

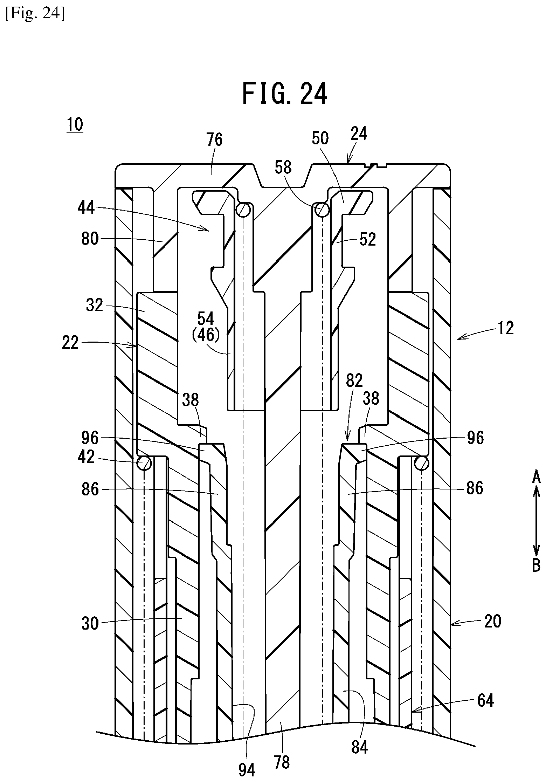

On completion of the puncture, along with the movement of the casing 12, the lock pin 44 moves relatively toward the proximal end (in direction of arrow A) as illustrated in FIGS. 21 and 22. Due to the relative movement, the small diameter portion 54 of the lock pin 44 is separated from the inside of the flexible portions 86 of the plunger rod 82 toward the proximal end (in direction of arrow A). Furthermore, the plunger rod 82 is biased toward the distal end (in direction of arrow B) by the resilience of the injection spring 58 and moves toward the distal end, and the four collars 96 are pushed radially inward under action of contact between the tapered distal end faces of the collars 96 and the protruding portions 38, causing the flexible portions 86 to be inclined radially inward relative to the pin body 46 and the collars 96 to move toward the distal end over the protruding portions 38 as illustrated in FIGS. 23 and 24.

In addition, the plunger rod 82 moves axially toward the distal end without rotating under action of guiding of the guide ribs 34 of the sleeve body 22 in which the four slide grooves 92 are inserted, and the gasket 106 mounted on the top 88 at the distal end is inserted in the barrel 124 of the syringe 16.

At this time, as illustrated in FIG. 6, when viewed from the axial directions of the plunger rod 82 and the sleeve body 22, the first clearance Cr1 is formed between each guide rib 34 and each slide groove 92 in the extending direction of the guide ribs 34, and the second clearance Cr2 is formed in the direction perpendicular to the extending direction.

Specifically, in FIG. 6, when two guide ribs on the right and left of the paper are denoted by 34a, and two guide ribs on the upper and lower side of the paper are denoted by 34b, the radial movements of the guide ribs 34a relative to the slide grooves 92 are suppressed by the guide ribs 34b disposed perpendicular to the guide ribs 34a, and the radial movements of the guide ribs 34b relative to the slide grooves 92 are suppressed by the guide ribs 34a disposed perpendicular to the guide ribs 34b. Furthermore, widths of the guide ribs 34a and 34b along the width direction perpendicular to the extending direction are designed to be smaller than a diameter of a piston in a conventional medication administration device. Accordingly, it is possible to make a dimensional tolerance smaller than that in the conventional piston. A smaller dimensional tolerance preferably reduces variation in radial position (eccentricity) of the plunger rod 82 relative to the sleeve body 22.

Therefore, the eccentricity of the plunger rod 82 to the sleeve body 22 is preferably suppressed, and what is more, the rotation of the plunger rod 82 relative to the sleeve body 22 is suppressed. With such a configuration, the plunger rod 82 is moved straight toward the distal end (in direction of arrow B) while being maintained substantially coaxial with the casing 12 (sleeve body 22).

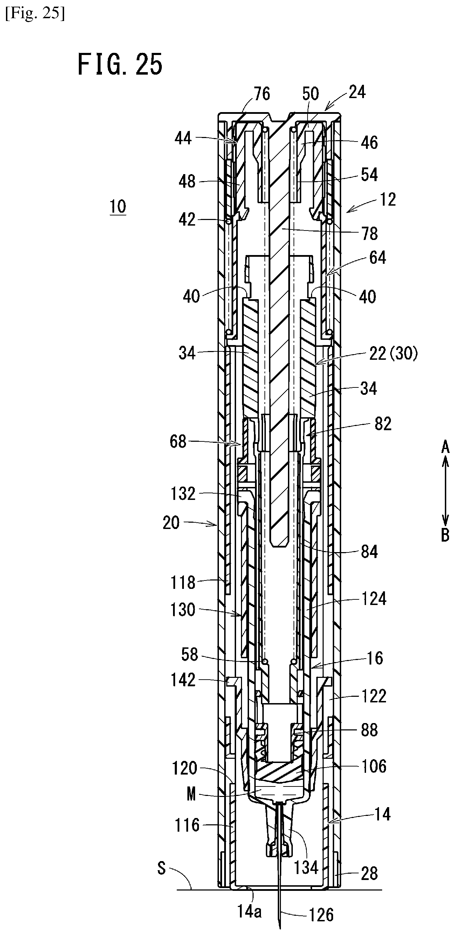

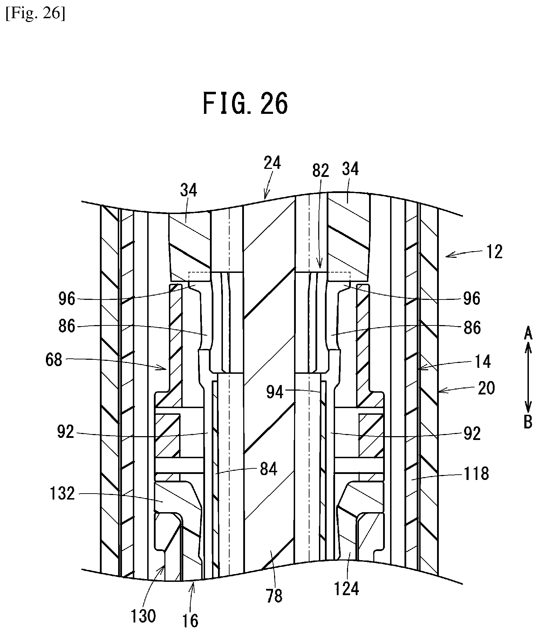

As illustrated in FIGS. 25 and 26, the movement of the plunger rod 82 toward the distal end (in direction of arrow B) causes the gasket 106 to press the medication M in the barrel 124 toward the distal end, thereby ejecting the medication M from the puncture needle 126 and administering it subcutaneously to the patient.

When the administration of the medication M is started along with the movement of the plunger rod 82, after the collars 96 of the plunger rod 82 move toward the distal end (in direction of arrow B) over the protruding portions 38 of the sleeve body 22 as illustrated in FIG. 24, the collars 96 spread radially outward again due to the elasticity that biases the collars 96 radially outward, and outer edges of the collars 96 touch the inner peripheral surface of the body main part 30. Since the outer diameter D1 of the collars 96 is formed slightly larger than the inner diameter D2 of the body main part 30 in the sleeve body 22 (see FIG. 8), when the collars 96 touch the body main part 30, a first informing sound or a contact sound (clink) is generated. This first informing sound allows the patient to confirm that the administration of the medication M is started.

After the administration of the medication M is started, the plunger rod 82 continuously moves toward the distal end (in direction of arrow B) at a constant speed by the resilience of the injection spring 58, and the gasket 106 moves toward the distal end inside the barrel 124 to push out the medication M and eject the medication M from the puncture needle 126. With this further movement of the plunger rod 82 toward the distal end, the flexible portions 86 reach a proximal end of the end guide 68, and the collars 96 touch the inclined guiding portions 72 as illustrated in FIG. 9A, causing the collars 96 to move toward the distal end (in direction of arrow B) while rotating clockwise along the inclined guiding portions 72. In other words, the plunger rod 82 moving along the inclined guiding portions 72 of the end guide 68 applies a rotational force to the plunger rod 82.

Simultaneously, the rotation of the plunger rod 82 causes the engagement sections 98 at the distal end to move along the horizontal portions 110 in the leading grooves 108 of the top 88 as illustrated in FIG. 9B. As illustrated in FIG. 25, the plunger rod 82 reaches the distal ends of the guide ribs 34 of the sleeve body 22 and is free from being axially guided by the slide grooves 92 and the guide ribs 34, thereby being rotatable.

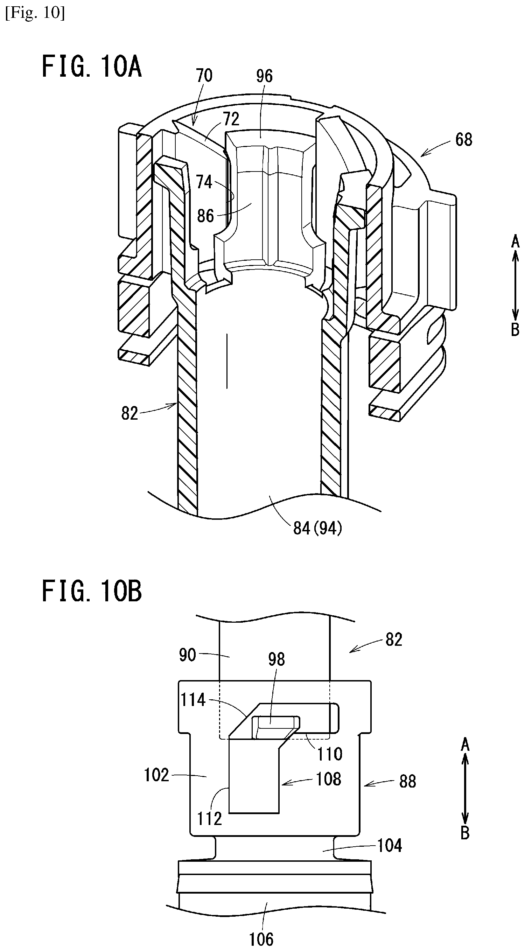

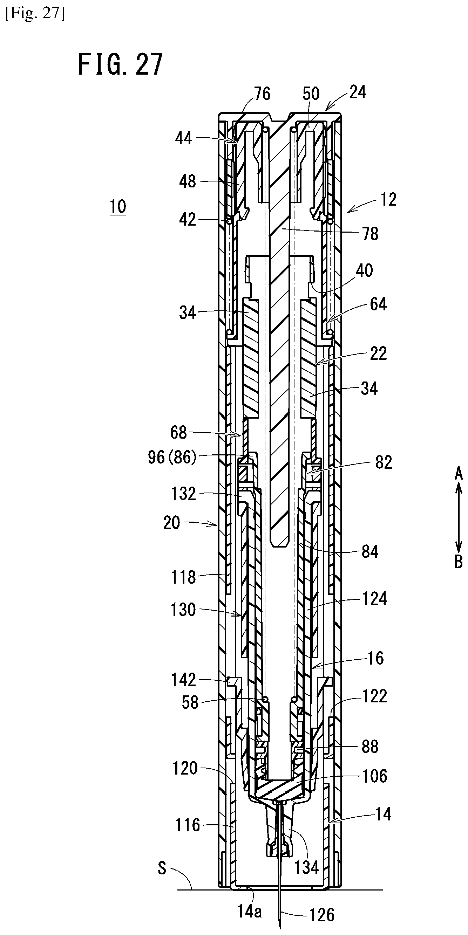

When the flexible portions 86 move toward the distal end and reach the straight guiding portions 74 while rotating along the inclined guiding portions 72 as illustrated in FIG. 10A, a predetermined dose of the medication M in the barrel 124 set in advance is administered to the skin S by the plunger rod 82 through the puncture needle 126 as illustrated in FIG. 27, thereby completing the administration of the medication M. Furthermore, before the completion of administration of the medication M, the engagement sections 98 of the rotating plunger rod 82 reach the inclined portions 114 of the leading grooves 108 as illustrated in FIG. 10B. As described above, the guide grooves 70 function as a guider that linearly moves the plunger rod 82 toward the distal end (in direction of arrow B) after rotating the plunger rod by a predetermined amount.

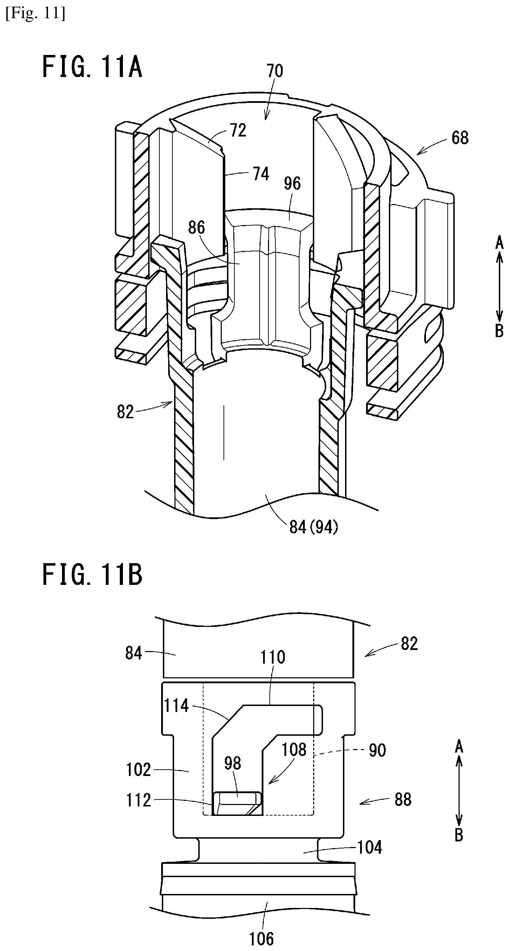

On completion of administration of the medication M, the engagement sections 98 of the plunger rod 82 move toward the perpendicular portions 112 while rotating along the inclined portions 114, and the engagement sections 98 are guided and moved toward the distal end along the perpendicular portions 112. This plunger rod 82 axially moves again toward the distal end (in direction of arrow B), and distal end faces of the engagement sections 98 moved along the perpendicular portions 112 of the leading grooves 108 touch distal edges of the perpendicular portions 112 as illustrated in FIG. 11B, thereby causing a second informing sound or a contact sound (clink).

The second informing sound is issued a predetermined time after the completion of administration of the medication M, allowing the patient to confirm that the medication administration to the punctuation target (skin S) is completed. After identifying the second informing sound, the patient separates the medication administration device 10 from the skin.

In other words, after the medication is completely administered by the medication administration device 10, the guide grooves 70 of the end guide 68 rotate the plunger rod 82 and decelerate an axial moving speed of the plunger rod 82 so as to delay the time when the gasket 106 of the top 88 connected to the plunger rod 82 reaches the distal end of the barrel 124, and after a predetermined time from the completion of the medication administration, the second informing sound is generated to give information on the completion of the medication administration.

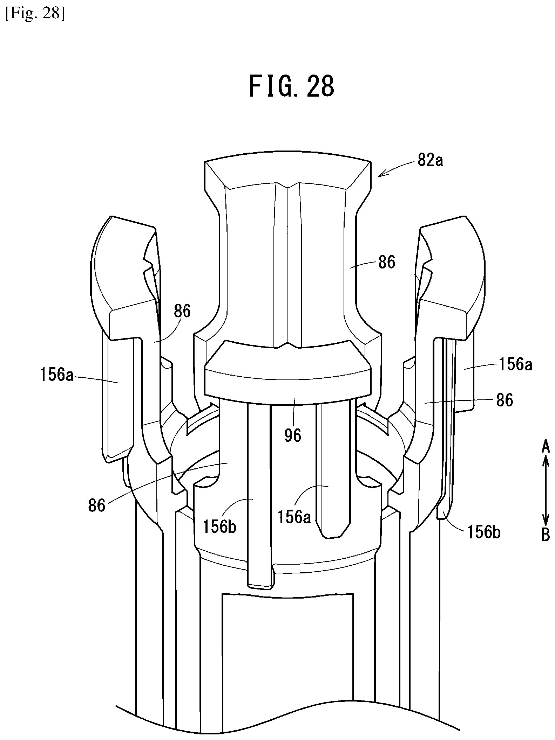

Alternatively, like a plunger rod 82a illustrated in FIGS. 28 to 29B, two first and second contact ribs 156a and 156b may be disposed on an outer peripheral surface of each flexible portion 86, extending axially from each collar 96 toward the distal end (in direction of arrow B), and protruding radially outward from the outer peripheral surface. A length of the first contact rib 156a is formed shorter than a length of the second contact rib 156b, so that a distal end of the first contact rib 156a is closer to each collar 96 than a distal end of the second contact rib 156b. Furthermore, the second contact rib 156b is substantially in parallel to the first contact rib 156a while being separated from the first contact rib 156a at a predetermined interval at a position in a direction of rotation of the plunger rod 82a. The number of contact ribs is not limited to two.

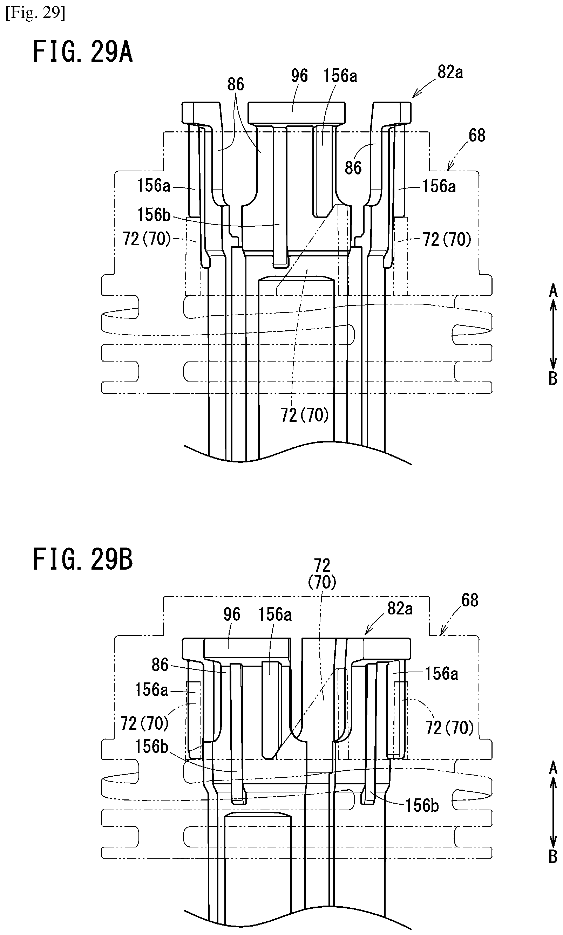

Accordingly, as illustrated in FIG. 29A, the distal ends of the first and second contact ribs 156a and 156b reach the proximal end of the end guide 68 and the distal ends touch the inclined guiding portions 72 of the guide grooves 70 along with the movement of the plunger rod 82a toward the distal end (in direction of arrow B), causing the plunger rod 82a including the first and second contact ribs 156a and 156b to move toward the distal end (in direction of arrow B) while rotating clockwise along the inclined guiding portions 72 (see FIG. 29B).

In other words, the first and second contact ribs 156a and 156b moving along the inclined guiding portions 72 of the end guide 68 applies a rotational force to the plunger rod 82a. An imaginary line connecting the distal end of the first contact rib 156a and the distal end of the second contact rib 156b is substantially parallel to the inclined guiding portions 72.

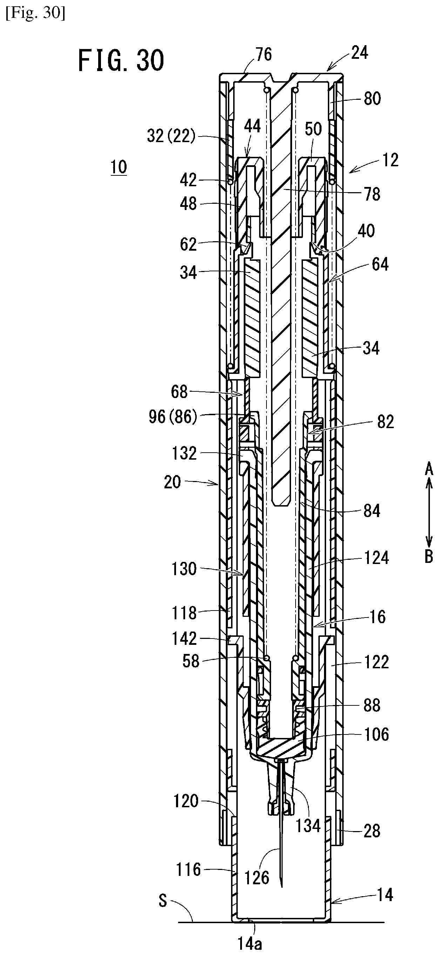

When a pressure on the medication administration device 10 toward the skin S is released, as illustrated in FIG. 30, the cover sleeve 14 is biased by the resilience of the sleeve spring 42 and moved toward the distal end (in direction of arrow B) relative to the sleeve body 22 of the casing 12, and the distal end of the sleeve body 22 is moved to a position closer to the distal end than the puncture needle 126 so as to cover the puncture needle 126.

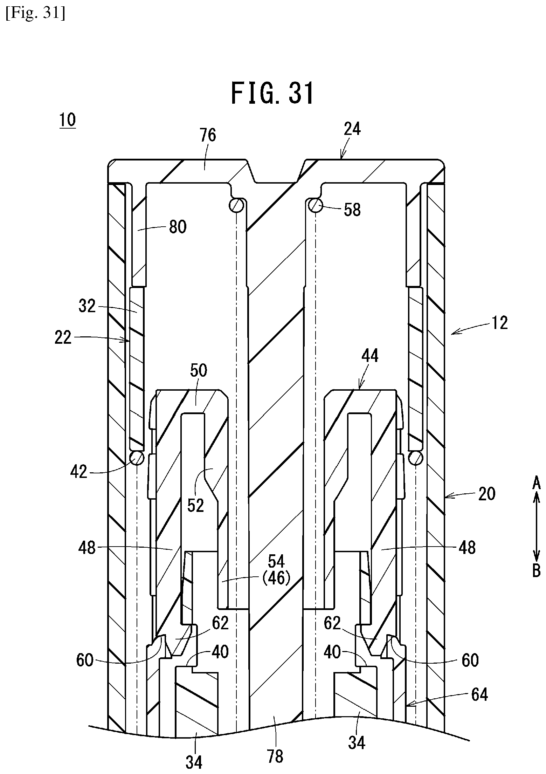

In addition, as illustrated in FIGS. 30 and 31, the lock pin 44 moves toward the distal end (in direction of arrow B) together with the cover sleeve 14, and the lock claws 62 are engaged with the lock grooves 40 of the sleeve body 22. Accordingly, the axial movement of the lock pin 44 relative to the sleeve body 22 is restricted, which results in restricting the movement toward the proximal end (in direction of arrow A) of the slide sleeve 64 abutting on the distal end of the sleeve body 22 and of the cover sleeve 14 abutting on the distal end of the slide sleeve 64.

With such a configuration, as illustrated in FIGS. 30 and 31, after the medication administration is completed and the patient separates the medication administration device 10 from the skin S, the puncture needle 126 is entirely covered by the cover sleeve 14. Furthermore, since the movement of the cover sleeve 14 toward the proximal end is restricted, the cover sleeve 14 prevents the puncture needle 126 from being exposed to the outside, which solves a safety problem.

As described above, in this embodiment, the axially movable plunger rod 82 (in directions of arrows A and B) is housed in the casing 12 included in the medication administration device 10, and an inner peripheral surface of the sleeve body 22 facing an outer peripheral surface of the plunger rod 82 is provided with the plurality of guide ribs 34 protruding radially inward. On the other hand, the outer peripheral surface of the plunger rod 82 is provided with the plurality of slide grooves 92 recessed radially inward and formed axially. In the slide grooves 92, the guide ribs 34 are inserted. At least three guide ribs 34 and slide grooves 92 are formed axially, being disposed along a circumferential direction of the sleeve body 22 and the plunger rod 82. Furthermore, the first clearance Cr1 is formed between each guide rib 34 and each slide groove 92 in the extending direction of the guide ribs 34, and the second clearance Cr2 is formed in the direction perpendicular to the extending direction.

With such a configuration, compared with the conventional medication administration device that secures the straightness with respect to the casing by controlling a diameter of the piston, it is possible to reduce dimensional tolerances by making widths of the guide ribs 34 and the slide grooves 92 smaller than the diameter of the piston, and it is possible to preferably suppress variation in radial position

(eccentricity) of the plunger rod 82 relative to the sleeve body 22, thereby enhancing the straightness.

For this reason, in the casing 12, when the plunger rod 82 is biased and moved toward the distal end by the resilience of the injection spring 58, the plunger rod 82 is guided along the guide ribs 34 inserted in the slide grooves 92, causing the plunger rod 82 not to rotate relative to the casing 12 and to be movable in a straight line toward the distal end (in direction of arrow B).

Accordingly, in the medication administration device 10, suppressing the eccentricity of the plunger rod 82 to the casing 12 and enhancing the straightness make it possible to move the plunger rod 82 smoothly toward the distal end and to stably perform administration of the medication and generation of the first and second informing sounds.

Furthermore, each of the guide ribs 34 has the ridge 36 inserted in each slide groove 92 and protruding in the width direction perpendicular to the extending direction at the end along the extending direction. Therefore, when the plunger rod 82 moves axially along the guide ribs 34, only the ridges 36 touch the slide grooves 92 and thus it is possible to reduce contact resistance as compared with a case where, for example, the entire end surfaces of the guide ribs 34 in the width direction touch the slide groove 92. Accordingly, the sliding resistance is suppressed when the plunger rod 82 moves axially along the casing 12, and the plunger rod 82 moves more smoothly toward the distal end.

Furthermore, the plurality of guide ribs 34 disposed at regular intervals along the circumferential direction of the plunger rod 82 makes it possible to suppress the eccentricity of the plunger rod 82 to the casing 12 more preferably to make the plunger rod 82 coaxial with the casing 12.

Furthermore, in the medication administration device 10, the four slide grooves 92 are disposed on the outer peripheral surface of the plunger rod 82 (rod body 84), and the four guide ribs 34 protruding radially inward and inserted in the slide grooves 92 are disposed on the inner peripheral surface of the sleeve body 22 (body main part 30) facing the outer peripheral surface. However, contrary to the above configuration, the guide ribs 34 may protrude radially outward on the outer peripheral surface of the plunger rod 82, and the slide grooves 92 into which the guide ribs 34 are inserted may be formed on the inner peripheral surface of the sleeve body 22.

## Claims

1. A medication administration device (10) for administering a medication to a living body, the medication administration device (10) comprising:
a casing (12) having a hollow cylindrical shape;
a syringe (16) including a barrel (20) housed in the casing (12) and filled with the medication and a puncture needle (126) communicated with the barrel (20) and configured to administer the medication to the living body;
a needle cover (64) having a hollow cylindrical shape, disposed inside the casing (12), covering a distal end of the syringe (16), and configured to be displaced toward a proximal end relative to the casing (12) when the needle cover (64) is pressed against a puncture target;
a plunger rod (82) disposed inside the casing (12), and configured to eject the medication through the puncture needle (126) by moving a distal end of the plunger rod (82) inside the barrel (20); and
a cap (18) detachably disposed at a distal end of the needle cover (64),
wherein one of an inner peripheral surface of the casing (12) and an outer peripheral surface of the plunger rod (82) facing the inner peripheral surface includes a plurality of guide ribs (34) protruding toward another of the inner peripheral surface of the casing (12) and the outer peripheral surface of the plunger rod (82),
the other of the inner peripheral surface of the casing (12) and the outer peripheral surface of the plunger rod (82) includes a guide groove (92) into which at least a part of the guide ribs (34) is inserted and which extends in a moving direction of the plunger rod (82), and
the guide ribs (34) are formed axially, the number of the guide ribs (34) being at least three, and
the guide ribs (34) including, between the guide ribs (34) and the guide groove (92), a first clearance (Cr1) formed in an extending direction of the guide ribs (34) and a second clearance (Cr2) formed in a direction perpendicular to the extending direction of the guide ribs (34), wherein the guide ribs (34) have an end along the extending direction of the guide ribs (34), inserted in the guide groove (92) and provided with a protruding portion (36) protruding in a direction substantially perpendicular to the extending direction of the guide ribs (34).

2. The medication administration device (10) according to claim 1,
wherein the protruding portion (36) is gradually tapered in a direction toward the inner walls of the guide groove (92) substantially perpendicular to the extending direction of the guide ribs (34).

3. The medication administration device (10) according to any one of claims 1 to 2,
wherein the guide ribs (34) are disposed at regular intervals in a circumferential direction of the plunger rod (82).

## Patentansprüche

1. Vorrichtung (10) zur Verabreichung von Medikamenten für die Verabreichung eines Medikaments an einen lebenden Körper, wobei die Vorrichtung (10) zur Verabreichung von Medikamenten umfasst:
ein Gehäuse (12), das eine hohle zylindrische Form aufweist;
eine Spritze (16), die einen Zylinder (20), welcher in dem Gehäuse (12) untergebracht und mit dem Medikament gefüllt ist, und eine Punktionsnadel (126) umfasst, die mit dem Zylinder (20) in Verbindung steht und konfiguriert ist, um das Medikament dem lebenden Körper zu verabreichen;
eine Nadelabdeckung (64), die eine hohle zylindrische Form aufweist, welche in dem Inneren des Gehäuses (12) angeordnet ist, ein distales Ende der Spritze (16) abdeckt und so ausgebildet ist, dass sie relativ zu dem Gehäuse (12) in der Richtung eines proximalen Endes verschoben wird, wenn die Nadelabdeckung (64) gegen ein Punktionsziel gedrückt wird;
eine Kolbenstange (82), die in dem Inneren des Gehäuses (12) angeordnet ist und so ausgebildet ist, dass sie das Medikament durch die Punktionsnadel (126) ausstößt, indem ein distales Ende der Kolbenstange (82) in dem Inneren des Zylinders (20) bewegt wird; und
ein Kappe (18), die abnehmbar an einem distalen Ende der Nadelabdeckung (64) vorgesehen ist,
wobei eine von einer inneren Umfangsfläche des Gehäuses (12) und einer äußeren Umfangsfläche der Kolbenstange (82), welche der inneren Umfangsfläche zugewandt ist, eine Vielzahl von Führungsrippen (34) aufweist, die in Richtung der anderen von der inneren Umfangsfläche des Gehäuses (12) und der äußeren Umfangsfläche der Kolbenstange (82) hervorstehen,
wobei die andere von der inneren Umfangsfläche des Gehäuses (12) und der äußeren Umfangsfläche der Kolbenstange (82) eine Führungsnut (92) aufweist, in die mindestens ein Teil der Führungsrippen (34) eingeführt ist und die sich in einer Bewegungsrichtung der Kolbenstange (82) erstreckt, und
die Führungsrippen (34) axial ausgebildet sind, wobei die Anzahl der Führungsrippen (34) mindestens drei beträgt und die Führungsrippen (34), zwischen den Führungsrippen (34) und der Führungsnut (92), einen ersten Zwischenraum (Cr1), der in einer Erstreckungsrichtung der Führungsrippen (34) ausgebildet ist, und einen zweiten Zwischenraum (Cr2), der in einer Richtung senkrecht zur Erstreckungsrichtung der Führungsrippen (34) ausgebildet ist, aufweisen,
wobei die Führungsrippen (34) ein Ende entlang der Erstreckungsrichtung der Führungsrippen (34) aufweisen, das in die Führungsnut (92) eingeführt ist und mit einem Vorsprungsabschnitt (36) versehen ist, der in einer Richtung im Wesentlichen senkrecht zu der Erstreckungsrichtung der Führungsrippen (34) hervorsteht.

2. Vorrichtung (10) zur Verabreichung von Medikamenten nach Anspruch 1,
wobei der Vorsprungsabschnitt (36) in einer Richtung zu den Innenwänden der Führungsnut (92) hin, welche im Wesentlichen senkrecht zu der Erstreckungsrichtung von den Führungsrippen (34) verläuft, sich allmählich verjüngt.

3. Vorrichtung (10) zur Verabreichung von Medikamenten nach einem der Ansprüche 1 bis 2,
wobei die Führungsrippen (34) in regelmäßigen Abständen in einer Umfangsrichtung der Kolbenstange (82) angeordnet sind.

## Revendications

1. Dispositif (10) d'administration de médicament pour administrer un médicament à un corps vivant, le dispositif (10) d'administration de médicament comprenant :
un boîtier (12) ayant une forme cylindrique creuse ;
une seringue (16) comportant un cylindre (20) logé dans le boîtier (12) et rempli du médicament et une aiguille de ponction (126) communiquant avec le cylindre (20) et conçue pour administrer le médicament au corps vivant ;
un cache d'aiguille (64) de forme cylindrique creuse, disposé à l'intérieur du boîtier (12), recouvrant une extrémité distale de la seringue (16), et conçu pour être déplacé vers une extrémité proximale par rapport au boîtier (12) lorsque le cache d'aiguille (64) est appuyé contre une cible de ponction ;
une tige de piston (82) disposée à l'intérieur du boîtier (12), et conçue pour éjecter le médicament à travers l'aiguille de ponction (126) en déplaçant une extrémité distale de la tige de piston (82) à l'intérieur du cylindre (20) ; et
un capuchon (18) disposé de manière détachable à une extrémité distale du cache d'aiguille (64),
dans lequel l'une d'une surface périphérique interne du boîtier (12) et d'une surface périphérique externe de la tige de piston (82) faisant face à la surface périphérique interne comporte une pluralité de nervures de guidage (34) faisant saillie vers une autre de la surface périphérique interne du boîtier (12) et de la surface périphérique externe de la tige de piston (82),
l'autre de la surface périphérique interne du boîtier (12) et de la surface périphérique externe de la tige de piston (82) comporte une rainure de guidage (92) dans laquelle est insérée au moins une partie des nervures de guidage (34) et qui s'étend dans une direction de déplacement de la tige de piston (82), et
les nervures de guidage (34) sont formées axialement, le nombre des nervures de guidage (34) étant d'au moins trois, et
les nervures de guidage (34) comportant, entre les nervures de guidage (34) et la rainure de guidage (92), un premier dégagement (Cr1) formé dans une direction d'extension des nervures de guidage (34) et un deuxième dégagement (Cr2) formé dans une direction perpendiculaire à la direction d'extension des nervures de guidage (34),
dans lequel les nervures de guidage (34) présentent une extrémité le long de la direction d'extension des nervures de guidage (34), insérées dans la rainure de guidage (92) et pourvues d'une partie saillante (36) faisant saillie dans une direction sensiblement perpendiculaire à la direction d'extension des nervures de guidage (34).

2. Dispositif (10) d'administration de médicament selon la revendication 1,
dans lequel la partie saillante (36) est progressivement effilée dans une direction vers les parois intérieures de la rainure de guidage (92) sensiblement perpendiculaire à la direction d'extension des nervures de guidage (34).

3. Dispositif (10) d'administration de médicament selon l'une quelconque des revendications 1 et 2,
dans lequel les nervures de guidage (34) sont disposées à intervalles réguliers dans une direction circonférentielle de la tige de piston (82).
